# EUROPEAN PATENT SPECIFICATION

(11) **EP 3 060 265 B1**
(45) Date of publication and mention of the grant of the patent: **27.11.2019**
(21) Application number: 14793730.4
(22) Date of filing: 23.10.2014
(51) Int. Cl.: A61L 26/00, A61L 24/00, A61L 27/20, A61L 24/08

(54) **CHITOSAN STENTING PASTE**
CHITOSAN-STENTING-PASTE
PÂTE D'OCCLUSION AU CHITOSANE

(30) Priority: 24.10.2013 US 201314061993; 30.06.2014 US 201414319901
(43) Date of publication of application: 31.08.2016
(73) Proprietor: Medtronic Xomed, Inc., Jacksonville, FL 32216-0980 (US)
(72) Inventor: MEDINA, Jennifer Gates, Jacksonville, FL 32216-0980 (US); SHERMAN, Ethan Glenn, Jacksonville, FL 32216-0980 (US)
(74) Representative: Dehns
(86) International application number: PCT/US2014/062029
(87) International publication number: WO 2015/061606

(56) References cited:
- WO-A1-96/13284
- WO-A1-99/07416
- WO-A1-2010/033943
- WO-A1-2011/060545
- WO-A2-02/40072
- CHELLADURAI SANKAR ET AL: "Design and Evaluation of Bioadhesive in-Situ Nasal Gel of Ketorolac Tromethamine", CHEMICAL & PHARMACEUTICAL BULLETIN, vol. 56, no. 11, 13 August 2008 (2008-08-13), pages 1596-1599, XP055159001, ISSN: 0009-2363, DOI: 10.1248/cpb.56.1596
- ATHANASIADIS THEO ET AL: "Effects of a novel chitosan gel on mucosal wound healing following endoscopic sinus surgery in a sheep model of chronic rhinosinusitis", THE LARYNGOSCOPE, WILEY-BLACKWELL, UNITED STATES, vol. 118, no. 6, 1 June 2008 (2008-06-01), pages 1088-1094, XP002535403, ISSN: 0023-852X, DOI: 10.1097/MLG.0B013E31816BA576
- Rowan Valentine ET AL: "The efficacy of a novel chitosan gel on hemostasis and wound healing after endoscopic sinus surgery", American Journal of Rhinology & Allergy, vol. 24, no. 1, 1 January 2010 (2010-01-01), pages 70-75, XP055450880, US ISSN: 1945-8924, DOI: 10.2500/ajra.2010.24.3422

## Description

### FIELD OF THE INVENTION

This invention relates to biomaterials for use in or on tissue and structures in the throat, nasal passages and elsewhere in or near the respiratory tract.

### BACKGROUND

Sinusitis is an inflammation of the mucosal tissue lining of the sinus walls which may lead to nasal passageway blockage, mucous stagnation and bacterial or fungal sinus cavity infection. Typical treatments begin with antibiotics. However, when antibiotics cannot relieve sinusitis, sinus surgery (which involves opening the sinus cavities and removing mucosal tissue) may be an alternative. Post-operative care for such surgery requires temporary and uncomfortable sinus packing or gauze which supports the reopened sinus passage and absorbs excess fluid while the tissues heal. After several days or at the discretion of the physician, the gauze packing is removed. Doing so is painful.

Rowan Valentine ET AL: "The efficacy of a novel chitosan gel on hemostasis and wound healing after endoscopic sinus surgery",American Journal of Rhinology & Allergy, vol. 24, no. 1, 1 January 2010 (2010-01-01), pages 70-75, discloses a chitosan/dextran crosslinked gel used after sinus surgery.

### SUMMARY OF THE INVENTION

The scope of the invention is defined by the claims. Any references in the description to methods of treatment refer to the products of the present invention for use in a method for treatment.

Sinus sealants and other biological materials have emerged as a promising technique to temporarily seal, stent or otherwise protect post-operative sinus passageways with less intrusion and pain than traditional packing techniques.

Packing or biomaterial stents that dissolve or otherwise degrade over a certain period are desirable. The packing or stents desirably should be able to be dispensed through a suitable dispensing device (for example, a syringe) using a single, gloved hand. Packing or stents should also desirably be provided as ready-to-use injectable or extrudable compositions.

The invention provides, in one aspect, a sinus stent paste as defined in claim 1.

The invention provides, in another aspect, a method as defined in claim 2, for preparing a chitosan stenting paste.

The invention provides in yet another aspect, a paste composition as defined in claim 3, for use in stenting internal tissue or an internal surgical site.

The disclosed paste, stent, method and use are especially useful for nasal and sinus surgery.

### BRIEF DESCRIPTION OF THE DRAWING

**Fig. 1** is a schematic view showing application of the disclosed paste to stent the middle turbinate.
**Fig. 2** and **Fig. 3** are perspective views respectively showing a syringe and bendable tip for use in dispensing the disclosed paste.

Like reference symbols in the various figures of the drawing indicate like elements. The elements in the drawing are not to scale.

### DETAILED DESCRIPTION

The following detailed description describes certain embodiments and is not to be taken in a limiting sense. All weights, amounts and ratios herein are by weight, unless otherwise specifically noted. The terms shown below have the following meanings:

The term "adhesion" refers to the sticking together of a body structure or prosthetic material to tissue, to the sticking together of tissue to tissue with which it is in intimate contact for an extended period, or to the formation of tissue that connects body structures, prosthetic materials or tissues to one another across a normally open space.

The term "antimicrobial" when used in reference to a substance means that the substance can kill, significantly inhibit or control the growth of microbes, for example bacteria such as *Staphylococcus aureus, Streptococcus epidermis, Pseudomonas aeruginosa* or *Escherichia coli.*

The term "biocompatible" when used in reference to a substance means that the substance presents no significant deleterious or untoward effects upon the body.

The term "biodegradable" when used in reference to a substance means that the substance will degrade or erode in vivo to form smaller chemical or physical species. Such degradation process may be enzymatic, chemical or physical.

The term "chitosan" refers to a polysaccharide polymer containing randomly distributed β-(1-4)-linked D-glucosamine (deacetylated) and optional N-acetyl-D-glucosamine (acetylated) monomer units, and includes chitosan derivatives in which one or more hydroxyl or amine groups of the polymer have been modified to alter the solubility or mucoadhesion characteristics of the derivative.

The term "conformal" when used in reference to a paste applied to tissue or other body structure means that the paste can form a substantially continuous layer over an area to which the paste has been applied.

The term "hemostat" means a device or material which stops blood flow.

The term "mucoadhesive" when used in reference to a substance means that the substance will adhere to the mucus covering epithelia.

The term "nasal or sinus" refers to the various tissues defining the normally air-filled passages and chambers within the nose and sinus cavities including but not limited to the nostrils or nares, the nasal concha or turbinates, the frontal, ethmoid, sphenoid and maxillary sinuses, the sinus ostia and the nasopharnyx.

The term "opaque" when used in reference to a material means that ordinary overhead illumination is not transmitted through about a 4 mm thick layer of the material.

The term "osmolality" means the number of osmoles of solute per kilogram of solvent, as measured using a freezing point depression osmometer.

The term "paste" when used in reference to a substance means the substance is a visibly homogenous, nonporous, opaque material having a soft, malleable, spreadable consistency, for example similar to toothpaste, and a viscosity such that the material is suitable for use in stenting (*e.g.,* holding apart) nasal or sinus tissue. An opaque gel may be a paste. A collection of free flowing dry solid particles, a non-malleable solid, a porous sponge, a translucent gel, a liquid or a sprayable composition would not be a paste.

The term "protective" when used in reference to a paste applied to tissue or other body structure means that the paste may assist in returning an injured, inflamed or surgically repaired tissue surface to a normal state, *e.g.,* through one or more healing mechanisms such as modulation of an inflammatory response, phagocytosis, mucosal remodeling, reciliation or other full or partial restoration of normal function.

The term "residence time" when used in reference to a paste applied to tissue or other body structure means the time period during which the paste or portion thereof remains in place *in vivo* under gross observation.

The term "room temperature" means a temperature of 20-25° C.

The term "thin" when used in reference to a protective layer atop tissue or other body structure means having an average thickness less than about five millimeters.

The term "tonicity" when used in reference to a cell's response (*e.g.,* ciliated tissue such as found in the ear, nose and throat) to an external substance refers to the sum of the concentration of solutes having the capacity to exert an osmotic force across a given membrane. Solutes that cannot cross the cell membrane exert an osmotic force. Depending on the solute concentration of the substance in reference to the cell membrane, tonicity may be referred to as "hypertonic", "hypotonic" or "isotonic". "Hypertonic" refers to a substance with a higher solute concentration outside a cell membrane. As such, when the substance contacts the cell membrane, water in the cell will have a tendency to move out of the cell to balance the solute concentration outside the cell membrane. "Hypotonic" refers to substance with a lower solute concentration outside the cell membrane. As such, water from outside the cell will enter into the cell, causing swelling in an attempt to balance the solute concentration inside the cell. "Isotonic" refers to a substance's solute concentration that is the same as the cell to which it comes in contact. As such, it is considered physiological with the cell and hence there is no net flow of water.

The term "viscosity" when used in reference to a substance is the extent to which the substance resists a tendency to flow when subjected to stress. Viscosity may be measured with a cone and plate viscometer that imposes a specific stress on the substance and the resultant stress deformation or resistance is measured according to ASTM F2103-11 (Part 5). The units of viscosity are reported as Pascal-seconds (Pa.s.). For the disclosed pastes, viscosity values are determined and reported after the paste has been sterilized.

The disclosed paste or method includes a high concentration of a water-soluble chitosan (*e.g.,* chitosan salt) and an osmolality reducing agent dissolved in a phosphate-containing solution, the paste having a pH of at least 4. The disclosed paste desirably has an off white to yellowish coloration, which makes it easy to visualize when applied. The disclosed paste is opaque. The disclosed paste is also desirably provided in a ready-to-use, storage-stable, injectable or extrudable form, requiring no or minimal preparation. The osmolality reducing agent is not a chitosan nor is the osmolality reducing agent crosslinkable with the chitosan. Because the paste does not include crosslinkers, it can be stored for extended time periods and desirably does not require further hydration, mixing or other similar preparation steps before application.

The disclosed paste may be prepared by mixing or dissolving the initially solid ingredients (*e.g.,* water-soluble chitosan and an osmolality reducing agent) in a phosphate-containing solution (*e.g.,* phosphate-buffered saline (PBS)). A paste is formed at room temperature (i.e. 20 °C to 25 °C) when the ingredients become solubilized and remains a paste at room temperature.

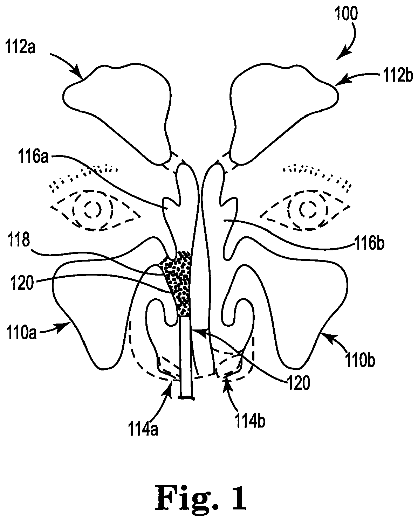

Referring to **Fig. 1****,** the disclosed paste or method may be used or performed for example in the nasal or sinus cavities **100** of a patient, including the maxillary sinuses **110a, 110b,** frontal sinuses **112a, 112b,** which may be accessed through nares **114a, 114b** and turbinates **116a, 116b.** It should be noted that external features of the patient, including nares **114a, 114b,** are shown in dashed lines. When the patient suffers for example from chronic rhinosinusitis, one or more treatment sites such as treatment site **118** associated with the middle turbinate **116a** may be medically or if need be surgically addressed. A layer **120** of the disclosed paste may be applied to site **118** and allowed to remain in place while healing takes place.

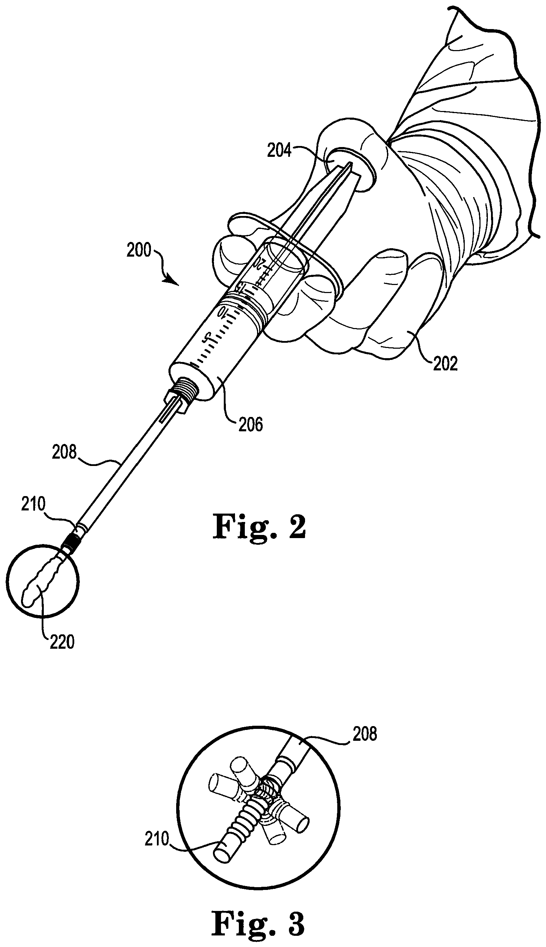

**Fig. 2** shows a dispensing syringe **200** for the disclosed paste held in the right hand **202** of a surgeon. When plunger **204** is depressed into syringe barrel **206,** the disclosed paste is dispensed through straight tip **208** and bendable tip **210,** whereupon it exits tip **210** as an opaque mass **220.** **Fig. 3** shows various bent positions (in phantom) that may be formed in bendable tip **210** to facilitate access to difficult to reach portions of a patient's anatomy.

Those skilled in the art will appreciate that the disclosed paste may be applied in or to other body parts. The disclosed paste may for example have utility in vascular or non-vascular applications, including treatment of tissues (*e.g.,* mucosal tissues) or other internal tissues or structures in or near the ears, throat, limbs or spinal column.

The applied paste may fill the treatment site (*e.g.,* a nasal or sinus cavity, or an opening, recess, passageway or joint in a portion of the limbs or spinal column), in which case the disclosed paste may be very thick and not exposed to air or other nearby gases, and with differing thicknesses throughout. The disclosed paste may also be applied as a thin film or other conformal coating in which case the layer may be relatively thin and exposed to air or other nearby gases, and with a substantially uniform thickness throughout the layer. The protective paste desirably adheres to mucosal or other natural tissues (*e.g.,* cartilage or bone) at the treatment site and resists detachment or other disruption until natural degradation or degradation initiated by irrigation or hydrolysis takes place, *e.g.,* after a residence time *in vivo* from at least 1 day, at least 3 days, at least 5 days, at least 7 days, at least 15 days, up to about 3 weeks, up to about 4 weeks, up to about 45 days or up to about 60 days. Meanwhile bacterial recolonization or reinfection may be significantly reduced or prevented, and improved healing (*e.g.,* reciliation) may take place. The protective paste may provide various therapeutic advantages including but not limited to bacterial adhesion inhibition, anti-infective properties, local immune modulation, tissue protection, reduction or elimination of pain or bleeding, reduction in inflammation, optimization of environment for ciliary regrowth, reduction in adhesions to critical anatomy, and the like. These advantages may arise due to a variety of mechanisms including a) killing bacteria, b) inhibiting bacterial colonization, c) inhibiting the adherence of bacteria to tissue, d) reducing tissue morbidity or abscess formation, e) reducing or preventing disease recurrence (for example, specifically reducing the chronic inflammation related to bacterial toxin and extracellular polysaccharide matrix (*viz*., biofilm) toxin), f) coating and protecting tissue during healing, such as by maintenance of a moist wound which promotes platelet aggregation, or by closure of a dry wound without excessive scabrous formation, g) hemostasis, h) optimizing the environment for reciliation of the mucosa, i) speeding the growth or regrowth of cilia and j) delivering therapeutic agent(s) to the treatment site.

Desirably the protective paste will adhere to a portion of the mucosa while leaving the cilia in unadhered portions free to undergo natural rhythmic cilia motion (*viz*., cilia beating), if desired also will deliver antimicrobial agents or additional therapeutic agents, and desirably will discourage or prevent bacteria from colonizing on the treatment site.

Water-soluble chitosans, preferably chitosan salts are used to form the paste. For example, high concentrations of a chitosan salt may be mixed in a phosphate-containing solution (*e.g.,* PBS, glycerol phosphate disodium salt hydrate or any combination thereof) to provide a ready-to use paste. The high chitosan salt concentration contributes both to the osmolality and opacity of the resulting paste. Without intending to be bound by theory, the phosphate and chitosan may react via an ionic reaction to help form the paste. The chitosan desirably is sufficiently water-soluble so that all of the desired chitosan amount can be dissolved in the disclosed paste. A portion of the chitosan may however be dispersed in the disclosed paste. The chosen chitosan preferably has a water solubility of at least about 3 wt. %, at least about 5 wt. %, at least about 8 wt. %, at least about 10 wt. %, at least about 15%, at least about 18% or at least about 20 wt. %. Exemplary chitosan concentrations from 5 to 20 wt. %, 5 to about 8 wt. %, about 8 wt. % to about 12 wt. %, about 12 wt. % to about 18 wt. %, about 16 wt. % to about 18 wt. % or about 18 wt. % to 20 wt. % of the total paste weight. The high chitosan concentrations used in the paste result in viscosities desirable for good stenting and acceptable syringe delivery force. Desired viscosities range from 1 to 15 Pa.s. when tested at 25° C and a shear rate of 221s⁻¹. This shear rate correlates to the approximate average shear rate the substance may experience as it is dispensed through a standard 30 ml BD™ syringe with a LUER LOCK™ connector at a rate of 1 ml/s.

Exemplary unmodified, water-soluble chitosans and their salts (including chloride, citrate, nitrate, lactate, phosphate, and glutamate salts) may be obtained from a variety of commercial sources including sources described in U.S. Patent Application Publication No. US 2009/0291911 A1.

Chitosan may also be synthesized by deacetylation of chitin (poly-N-acetyl-D-glucosamine) to eliminate acetyl groups on the nitrogen atom by hydrolysis. The resulting polymer has a plurality of repeating units (*e.g.,* about 30 to about 3000 repeating units, about 60 to about 600 repeating units, or such other amount as may be desired for the chosen end use) some or all of which contain deacetylated amino groups (*e.g.,* about 30 to about 100% or about 60 to about 95% of the total repeating units), with the remaining repeating units (if any) containing acetylated amino groups. The polymer is cationic and may be regarded as being composed from glucosamine monomers.

The chitosan may have a variety of number average molecular weights, *e.g*., about 5 to about 2000 kDa, about 10 to about 500 kDa, or about 10 to about 100 kDa. The chitosan may for example be an ultralow molecular weight material having a number average molecular weight less than or about 30 kDa, a low molecular weight material having a number average molecular weight of about 30 to about 400 kDa, a medium molecular weight material having a number average molecular weight of about 200 to about 500 kDa or a high molecular weight material having a number average molecular weight greater than about 500 kDa. A low molecular weight chitosan is preferred. The disclosed molecular weights are weights before sterilization of the paste. The chitosan desirably is in dry particulate form prior to mixing with the phosphate solution, for example, as free-flowing granules whose average particle diameter is less than about 1 mm, less than about 100 µm, about 1 to about 80 µm, or less than 1 µm.

Chitosan derivatives may also be employed, in which one or more hydroxyl or amino groups have been modified for the purpose of altering the solubility or mucoadhesion characteristics of the derivative. Exemplary derivatives include thiolated chitosans, and non-thiolated chitosan derivatives such as acetylated, alkylated or sulfonated chitosans (for example O-alkyl ethers, O-acyl esters, cationized trimethyl chitosans and chitosans modified with polyethylene glycol). Chitosan derivatives may be obtained from a variety of sources. For example, thiolated chitosans may be obtained from ThioMatrix Forschungs Beratungs GmbH and Mucobiomer Biotechnologische Forschungs-und Entwicklungs GmbH or prepared by reaction of chitosan with a suitable thiolated reactant, e.g., as described in Published PCT Application No. WO 03/020771 A1 or in Roldo et al., Mucoadhesive thiolated chitosans as platforms for oral controlled drug delivery: synthesis and in vitro evaluation, European Journal of Pharmaceutics and Biopharmaceutics, 57, 115-121 (2004); Krauland et al., Viscoelastic Properties of a New in situ Gelling Thiolated Chitosan Conjugate, Drug Development And Industrial Pharmacy, 31, 885-893 (2005); Bernkop-Schnürch, Thiomers: A new generation of mucoadhesive polymers, Advanced Drug Delivery Reviews, 57, 1569-1582 (2005); and Bernkop-Schnürch et al., Thiomers: Preparation and in vitro evaluation of a mucoadhesive nanoparticulate drug delivery system, International journal of Pharmaceutics, 317, 76-81 (2006).

While a high chitosan concentration has a desired viscosity for stenting, it also results in high osmolality (*e.g.,* a paste made from about 18 wt. % chitosan HCl and having a molecular weight in the range of about 30 to about 400 kDa results in an osmolality of about 2800 mOsm/kg). A high osmolality paste may not be physiologically compatible with the cells or tissues to which the paste may be applied. The desired osmolality is such that the paste is considered isotonic or hypertonic to the cells or tissues (*e.g.,* ciliated tissue) to which the paste is applied. The paste has an osmolality of from
270 to 2000 mOsm/kg or about 270 to about 1500 mOsm/kg, yet has a desirable viscosity.

To maintain or lower the osmolality without significantly altering or reducing the desired viscosity or the paste-like consistency, it was found that one or more osmolality reducing agents should be used. The osmolality reducing agent desirably is sufficiently water-soluble so that all of the desired amount of the osmolality reducing agent can be dissolved in the disclosed paste. A portion of the osmolality reducing agent may however be dispersed in the disclosed paste. The chosen osmolality reducing agent preferably has a water solubility of at least about 1 wt. %, at least about 2 wt. %, at least about 8 wt. %, at least about 10 wt. %, or at least about 20 wt. %. The osmolality reducing agent desirably contains few or no salt groups as such groups may increase rather than decrease the osmolality of the disclosed paste. Recommended amounts of the osmolality reducing agent may for example be about 1 to about 20 wt. %, about 1 to about 10 wt. % or about 2 to about 8% wt. of the total paste weight. Examples of suitable osmolality reducing agents include polysaccharides other than chitosans that are biocompatible and which reduce osmolality in the disclosed paste but which do not crosslink the chitosan. Examples of such polysaccharides include agars, alginates, carrageenans, celluloses, dextrans, galactomannans, glycogens, hyaluronic acids, and starches, and especially those that are water-soluble to the desired extent without containing salt groups.

Preferred polysaccharides include hydroxyl-functional or alkyl-modified celluloses. Exemplary cellulose materials include methylcellulose, ethylcellulose, hydroxybutyl methylcellulose, hydroxyethylcellulose, hydroxypropylcellulose, hydroxypropyl methylcellulose, carboxymethylcellulose, and mixtures thereof. Without intending to be bound by theory, it is believed that the osmolality reducing agent serves as a "salt scavenger" that may help reduce the paste's osmolality and maintain the desired paste-like consistency.

The paste may for example contain chitosan and an osmolality reducing agent in a combined amount representing about 1 to about 20 wt. %, about 10 to about 20 wt. % or about 10 to about 15 wt. % of the total paste composition. The chitosan and osmolality reducing agent may for example be combined in a ratio of about 10:1 to about 1:20, about 5:1 to about 1:10, or about 3:1 to about 1:5.

The paste has a pH appropriate for contacting human tissue, which is a pH of at least 4, a near-neutral pH, or a pH less than 10. An acid, base or buffering agent may for example be included to help maintain an appropriate pH. Buffering agents are preferred and phosphate-containing buffers are most preferred. Exemplary buffering agents include mixtures of barbitone sodium, glycinamide, glycine, potassium chloride, potassium phosphate, potassium hydrogen phthalate, sodium acetate, sodium citrate or sodium phosphate with their conjugate acids (for example a mixture of sodium citrate and citric acid).

Exemplary phosphate-containing buffers are derived from phosphoric acid and a base selected from potassium hydroxide, sodium hydroxide, the potassium or sodium salts of phosphoric acid, mixtures thereof and the like. Exemplary phosphate salts include sodium phosphate dibasic and monobasic, potassium phosphate dibasic and monobasic and mixtures thereof. The concentration of phosphoric acid and base or salt in the disclosed buffering agent may be varied to achieve the desired pH.

As discussed above, the chitosan and the osmolality reducing agent are dissolved in the phosphate-containing solution. Exemplary phosphate-containing solutions include phosphate-containing buffers such as PBS. PBS solutions typically include a combination of one or more phosphate salts and one or more chloride salts. Exemplary phosphate salts include disodium phosphate, potassium dihydrogen phosphate or a combination thereof. Exemplary chloride salts include sodium chloride, potassium chloride or a combination thereof. The salts used to prepare the PBS solution are optionally hydrates. An exemplary combination of salts employs disodium phosphate heptahydrate (Na₂HPO₄·7H₂O) and potassium dihydrogen phosphate (KH₂PO₄), in a so-called 1X PBS solution with concentrations of about 0.01M phosphate, about 0.0027M KC1, about 0.137 M NaCl and a pH of 7.4 at 25° C. PBS buffer solutions may be prepared in other strengths such as 2X, 3X, 5X, 10X or any other suitable strength. For example, a 10X PBS buffer may be prepared by adding 10 times the ingredients described for a 1X PBS buffer to result in concentrations of about 0.1M phosphate, about 0.027M KC1, and about 1.37 M NaCl. Preferably, the phosphate-containing solution is a PBS solution and more preferably is greater than a 1X PBS solution. Preferably, the PBS solution has a pH between about 9 to about 12, and more preferably is a 3X solution having a pH of about 11.

Phosphates may also be provided as salts of glycerol-3-phosphate (GlyPhos) (*e.g.,* as sodium, potassium, calcium or magnesium salts). Stereoisomeric forms of GlyPhos, preferably the racemic, meso, α and β blends or other forms or blends, may also be used. In some embodiments, the phosphate may be provided by a buffering agent (*e.g.,* PBS), by a salt of GlyPhos or both.

The disclosed paste may consist of or consist essentially of the above-mentioned water-soluble chitosan or derivative, osmolality reducing agent and phosphate-containing solution, optionally together with any one or more of a variety of adjuvants. Exemplary adjuvants include lubricants, wetting agents, therapeutic agents, antimicrobial agents, dyes, pigments or other colorants, indicators, flavoring or sweetening agents, antioxidants, antifoam agents, thixotropes, release agent modifiers for sustained or delayed release of therapeutic agents, and other ingredients that will be known to persons having ordinary skill in the art.

Lubricants and wetting agents are especially desirable adjuvants that may help maintain paste consistency, and may aid in dispensing the paste into or onto a desired treatment site. Desirably, the paste should be able to be dispensed by an operator from a suitable delivery device (for example a syringe) using a single gloved hand. One preferred class of lubricants and wetting agents includes hydroxy compounds having two or more hydroxyl groups with the presence of 1,2-diol grouping being desirable. Hydroxy compounds having 2-4 carbon atoms have been found to be particularly useful lubricants. Glycerol is especially preferred. Other compounds include ethane-1,2-diol; propane-1,2-diol; butane-1,3-diol and butane-1,4-diol. Mixtures of hydroxy compounds may be employed, especially mixtures of glycerol and one or more diols. Desired amounts of the lubricants and wetting agents may for example be about 1 to about 15 wt. % or about 2 to about 12 wt. % of the total paste weight.

Exemplary therapeutic agents include any material suitable for use at the intended treatment site including analgesics, anti-cholinergics, anti-fungal agents, antihistamines, steroidal or non-steroidal anti-inflammatory agents, anti-parasitic agents, antiviral agents, biostatic paste, chemotherapeutic agents, antineoplastic agents, cytokines, decongestants, hemostatic agents (*e.g.,* thrombin), immunosuppressors, mucolytics, nucleic acids, peptides, proteins, steroids, vasoconstrictors, vitamins, mixtures thereof, and other therapeutic materials that will be known to those skilled in the art. A useful list of such therapeutic agents may also be found, for example, in U.S. Patent No. 7,959,943 B2 (Hissong et al).

The disclosed paste desirably is inherently antimicrobial without requiring addition of a separate antimicrobial agent. Antimicrobial activity may be influenced by the proportion of chitosan in the paste. A separate antimicrobial agent may be employed if desired. A useful list of such antimicrobial agents may be found, for example, in U.S. Patent No. 7,959,943 B2.

Exemplary dyes, pigments or other colorants include FD & C Red No. 3, FD & C Red No. 20, FD & C Yellow No. 6, FD & C Blue No.2, D & C Green No. 5, D & C Orange No. 4, D & C Red No. 8, caramel, titanium dioxide, fruit or vegetable colorants such as beet powder or beta-carotene, turmeric, paprika and other materials that will be known to those skilled in the art). Exemplary indicators; flavoring or sweetening agents include anise oil, cherry, cinnamon oil, citrus oil (*e.g.,* lemon, lime or orange oil), cocoa, eucalyptus, herbal aromatics (*e.g.,* clove oil, sage oil or cassia oil), lactose, maltose, menthol, peppermint oil, saccharine, sodium cyclamate, spearmint oil, sorbitol, sucrose, vanillin, wintergreen oil, xylitol and mixtures thereof.

The disclosed paste typically will be placed in suitable sealed packaging (for example, a syringe, a vial, or pouch made of suitable materials, or a kit containing such packaging and optional printed instructions) and subjected to sterilization before being further packaged if need be with printed instructions describing the proper use of the paste or kit in nasal or sinus surgery. Sterilization methods that do not unduly discolor (*e.g.,* brown), affect the adhesive strength or viscosity or otherwise unduly affect the paste's consistency are desirable. Suitable sterilization methods include steam or ionizing radiation (*e.g.,* gamma radiation and electron beam (E-Beam)). E-Beam sterilization appears to prevent or limit paste discoloration. E-beam sterilization may be performed at reduced temperatures as described in U.S. Patent No. 8,653,319 B2 (Amery et al). E-beam or gamma sterilization may for example be used at doses in the range of about 12 to about 40 kGy. In some embodiments the disclosed paste may be translucent before sterilization and opaque after sterilization.

The paste desirably is provided as a ready-to-use composition requiring little or no mixing, stirring, hydration or other preparation. The paste desirably is provided in a dispenser from which the paste may be injected or extruded, and may for example be packaged in unit doses of about 5 to about 100 g. The paste desirably has good shelf life as determined by adhesive strength, viscosity and pH, and preferably may be stored for more than 12 months. In some embodiments, the paste may be stored for more than 15 months, more than 18 months or up to 24 months while still maintaining a viscosity of about 1 to about 15 Pa.s. If the paste appears to have separated, re-mixing (*e.g.,* moving the composition back and forth between two syringes) typically will return the paste to a more homogenous consistency. However, the paste preferably does not separate during storage. The paste desirably is stable at temperatures ranging from about 2° C to about 60° C. In addition, the paste desirably remains a paste after exposure to extreme temperature ranges imposed during ISTA-2A testing (*e.g.,* about -29°C to about 60°C).

The disclosed paste also may have desirable mucoadhesion. In other words, the disclosed paste preferably will adhere or stick to the specific body tissue or passageway to which it is applied without having to fully pack the passageway to obtain adequate retention in the passageway. Desirably, the paste has an adhesive strength such that a separation force of about 5 grams to about 80 grams, about 20 to about 50 grams or about 15 to about 30 grams may be required. The separation force may be measured as the force required when using a tensile testing machine (*e.g.,* an MTS™ tensile testing machine) operated at a separation rate of 1 mm/s to separate two collagen-coated, rubber hemispheres compressed against one another, with a sample of the paste between them, using about a 4.4 Newton (1 pound) compression force. The rubber hemispheres desirably are made from ultra soft Shore OO, 30 durometer black rubber balls with a diameter of about 5 cm (2 inches) that have been bisected to a height of about 2.5 cm (1 inch). The hemispheres desirably are mounted in the tensile tester in opposing convex relationship with a piece of sausage casing wrapped over each hemisphere. About 0.2 to about 0.5 ml of the disclosed paste desirably is applied to the center of the lower hemisphere, the hemispheres are compressed together using the specified compression force and the force required to separate the hemispheres at the specified separation rate is recorded. Adhesion strength values are reported for sterilized paste. The paste has a residence time in the applied passage or structure of at least 1 day, at least 3 days, at least 5 days, or at least 7 days with or without irrigation. The paste may degrade naturally or by irrigation (*e.g.,* saline solution).

The disclosed paste desirably is non-cytotoxic with cytotoxicity scores of 0, 1 or 2 as measured by ISO Guideline 10993-5, Biological Evaluation of Medical Devices-Part 5: Tests for *in vitro* Cytotoxicity. Desirably, the paste may have a cytotoxicity score of 1 or less.

The disclosed paste desirably is substantially collagen-free. Desirably the paste is sufficiently free of collagen (*e.g.,* containing no collagen at all) so as to be saleable worldwide for use without restriction in humans. The disclosed paste desirably does not contain other ingredients which might potentially harm mucosal tissues or structures, *e.g.,* tissues in the nasal or sinus cavities.

The disclosed paste may be used as a part of a multi-step treatment regimen. For example, a series of steps that may be broadly classified as Cleansing/Disrupting, Killing, Aerating, Protecting/Coating, and Healing may be carried out. The Cleansing/Disrupting step may be carried out by administering a solvating system like those described in U.S. Patent Nos. 7,976,873 B2 and 7,976,875 B2 and in U.S. Patent Application Publication No. 2011/0245757A1. The Killing step may be carried out by applying a suitable antimicrobial agent to the treatment site. This may for example be accomplished by including an antimicrobial agent in the solvating system, as a separately-applied agent, or in both the solvating system and the separately-applied agent. An antimicrobial agent may also be applied or administered post operatively. The Aerating step may be carried out by providing air passageways or improving air passageways to the treated tissues by opening occluded or partially occluded passages, *e.g.,* the sinuses or sinus ostia for nasal applications. This may for example be accomplished by surgically removing obstructive tissue structures or by manually displacing such structures. The Protecting/Coating step may be carried out by coating at least part of the thus-treated tissue with the disclosed paste. The Healing step may be carried out by allowing the cleansed, protected and sealed tissue surface to undergo a return to a normal state, *e.g.,* through one or more healing mechanisms such as modulation of an inflammatory response, phagocytosis, mucosal remodeling, reciliation or full or partial restoration of normal function. The multi-step treatment regimen may include or be followed by a Clearing step in which the disclosed paste is sufficiently biodegradable to disappear from the treatment site in a desired time period, *e.g.,* more than 5 days, or about 7 to 15 days or by irrigation. The paste desirably degrades or can be otherwise removed without shedding large solid chunks. The disclosed method may advantageously be accomplished without requiring surgery, for example by applying and removing the optional solvating system and by applying the disclosed paste through normal aspiration or suction techniques or by simple flushing of affected tissue.

The disclosed paste may be used for a variety of indications. Exemplary such indications include use as a stent or space occupying packing to separate tissue or structures compromised by surgical trauma, and to separate and prevent adhesions between mucosal surfaces in the nasal cavity. Other indications include the control via a tamponade effect, blood absorption or platelet aggregation of bleeding (*e.g.,* limited bleeding) and oozing of debrided mucosal surfaces following surgery or trauma, thereby aiding hemostasis; acting as an adjunct to aid in the natural healing process; acting as a nasal packing to treat epistaxis; and providing an antibacterial barrier or antibacterial effect.

The invention is further illustrated in the following non-limiting examples.

### Example 1

### Paste Formulations

A 3X PBS (pH 11-12) solution was prepared by dissolving PBS tablets in water and the pH was adjusted to pH 11-12 using IN NaOH. Glycerol if used was then added to the PBS solution to form a PBS/Glycerol solution. To either the PBS solution or PBS/Glycerol solution were added varying amounts of dry ingredients, namely a 30-400 kDa molecular weight chitosan, glycerol phosphate disodium salt hydrate solid or polysaccharide and mixed at room temperature to form a paste. The paste was then gamma sterilized. All formulations except for formulation 4 formed a paste. Formulation 4 was initially liquid-like and subsequently became sticky and stringy. Table 1 shows the percentage of the ingredients in the total volume of liquid. Table 1 also shows the osmolality, viscosity and adhesion values for each formulation after sterilization.

**Table 1**

| **Formulation** | **Chitosan HCl(%)** | **Glycerol (%)** | **BGlyPh (%)** | **Polysaccharide (%)** | **Osmolality (mOsm/kg)** | **Sterile Viscosity (Pa.s.) at Shear Rate 221 (1/s)** | **Average Sterile Adhesion Force (grams)** |
|---|---|---|---|---|---|---|---|
| **1** | 13 | 0.6 | | | 2087 | 1.9 | 26.6 |
| **2** | 17 | 0.6 | | | 1730 | 3.2 | 26.4 - 37.7 |
| **3** | 17 | 0.6 | 6 | | 2882 | 4.9 | 38.3 |
| **4** | 8.5 | | 2 | dextran (20%) | 329 | 6.1 | 19.3 |
| **5** | 8.5 | | 2 | hydroxyethyl cellulose (2.5%) | 495 | NA | Less than 5 |
| **6** | 8.5 | | 2 | methyl cellulose (10%) | 557 | 10.5 | 39.5 |
| **7** | 8.5 | | 2 | hydroxypropyl cellulose (15%) | 177 | 8.2 | 33.1 |
| **8** | 13 | 0.6 | | hydroxypropyl cellulose (10%) | 1492-2048 | 13.8 | 44.4 |
| **9** | 13 | 0.6 | | hydroxypropyl cellulose (5%) | 2172 | 4.9 | 38.7 |
| **10** | 13 | 0.6 | | hydroxypropyl cellulose (1%) | 2048-2067 | 1.6 | 24.3 |
| **11** | 17 | 0.6 | | hydroxypropyl cellulose (10%) | 2100 | 10.7 | 62.2 |
| **12** | 17 | 0.6 | | hydroxypropyl cellulose (5%) | 1787-1995 | 9.9 | 68.1 |
| **13** | 17 | 0.6 | | hydroxypropyl cellulose (1%) | 2083 | 3.5 | 35.4 |

### Example 2

0.6 ml of 10 % glycerol and 5.4 ml of 3X PBS solution (pH 11) as prepared in Example 1 were mixed in a 10 ml syringe. To this PBS/Glycerol solution was added varying amounts of glycerol phosphate, chitosan *HCl* and hydroxypropyl cellulose (HPC) in solid form. After all the ingredients in the syringe were fully mixed at room temperature, the resultant paste was gamma or E-beam sterilized. The formulations are shown below in Table 2. All the formulations formed a paste at room temperature and remained a paste at room temperature. Table 2 shows the percentage of the various ingredients reported as a percentage of the total volume of liquid. The osmolality, viscosity and adhesion values for each formulation are shown below in Table 3 and are values after sterilization.

**Table 2**

| **Formulation** | **Chitosan HCl (%)** | **HPC (%)** | **Glycerol Phosphate (%)** |
|---|---|---|---|
| **14** | 8.5 | 3 | 1 |
| **15** | 13 | 4 | 2 |
| **16** | 10 | 3 | 1 |
| **17** | 10 | 3 | 1.5 |
| **18** | 13 | 2 | 2 |

**Table 3**

| **Formulation** | **Osmolality (mOsm/kg)** | **Sterile Viscosity (Pa.s.) at Shear Rate 221 (1/s) Gamma** | **Sterile Viscosity (Pa.s.) at Shear Rate 221 (1/s) E-Beam** | **Sterile Adhesion Force (grams) Gamma** | **Sterile Adhesion Force (grams) E-Beam** |
|---|---|---|---|---|---|
| **14** | Less than 1492 | 3 | 3.2 | 13.5 | 40.6 |
| **15** | Greater than or equal to 1492 | 1.7 | 0.4 | 14.5 | 45.5 |
| **16** | 525 | 1.8 | 2.3 | 22.8 | 65.0 |
| **17** | Less than 1492 | 1.6 | 1.4 | 17.4 | 67.3 |
| **18** | Greater than or equal to 1492 | 2.2 | 3.2 | 19.8 | 50.5 |

### Example 3

### Antimicrobial Properties

Formulations 14, 15 and 16 from Table 2 were evaluated to determine their antimicrobial activity against four common bacterial strains (*S. aureus, S. epidermis, E. coli* and *P. aeruginosa* using a zone of inhibition screening technique.

The four bacteria were grown on Muller Hinton agar plates. Under sterile conditions, approximately 0.1 to 0.2 ml of each formulation was directly placed on the agar plates. The agar plates were incubated at 35 °C for 12 hours. After incubation, the plates were observed for bacterial growth. The use of the term "zone of inhibition" denotes an area around the formulations where bacterial growth was inhibited. The term "bacteriostatic" denotes that the bacteria grew to the edge of the formulation but no further growth was observed. In other words, the term "bacteriostatic" refers to an ability to prevent bacteria from growing and multiplying but possibly not killing them.

The results shown in Table 4 below are based on triplicates per formulation.

**Table 4**

| **Antimicrobial Properties** | | | |
|---|---|---|---|
| | **Zone of Inhibition or Bacteriostatic** | | |
| **Bacterial Strains** | **Formulation 14** | **Formulation 15** | **Formulation 16** |
| *S. aureus* | zone of inhibition | zone of inhibition | zone of inhibition |
| *S. epidermis* | zone of inhibition | zone of inhibition | zone of inhibition |
| *E. coli* | zone of inhibition | zone of inhibition | zone of inhibition |
| *P. aeruginosa* | bacteriostatic | bacteriostatic | bacteriostatic |

The results show that the formulations were antimicrobial and produced zones of inhibition.

Formulation 16 was evaluated according to United States Pharmacopeia (USP) Guidelines for Performing Antimicrobial Effectiveness Testing, Chapter <51> and Validation of Microbial Recovery, Chapter <1227> to determine antibacterial efficacy against common organisms typically found in treatment application areas. The measured antimicrobial properties are shown below in Table 5:

**Table 5**

| **Antimicrobial Properties**, **USP** | | | | | | |
|---|---|---|---|---|---|---|
| **Bacterial strain** | **ATCC No.** | **Gram stain** | **Antibacterial -Log Reduction** | | | |
| | | | **1 hour** | **24 hours** | **3 days** | **7 days** |
| *Pseudomonas aeruginosa* | 9027 | neg | 3 | 4.8 | 5.3 | >5.3 |
| *Staphylococcus aureus* | 25923 | pos | 0 | 2.7 | 4 | >5.0 |
| *Staphylococcus epidermidis* | 12228 | pos | 1.2 | 5.1 | 5 | >5.3 |
| *Echerichia coli* | 25922 | neg | 0 | 1.6 | 3.9 | >5.1 |
| *Citrobacter freundii* | 8090 | neg | N/A | 4.7 | 5 | >5.3 |
| *Enterobacter aerogenes* | 13048 | neg | N/A | 2 | 2.3 | >5.1 |
| *Klebsiella pneumonia* | 4352 | neg | N/A | 4 | 4 | >5.2 |
| *Proteus mirabilis* | 4630 | neg | N/A | 2 | 4 | >5.3 |
| *Serratia marcescens* | 13880 | neg | N/A | 1.5 | 2.8 | >5.0 |
| *Haemophilus influenzae* | 53782 | neg | N/A | >4.9 | >4.9 | >4.9 |
| *Moraxella catarrhalis* | 8193 | neg | N/A | 2.9 | 4 | >5.0 |
| *Staphylococcus aureus(MRSA)* | 33591 | pos | N/A | 0.6 | 3.7 | >5.1 |
| *Staphylococcus saprophyticus* | 15305 | pos | N/A | 4.6 | 4.9 | >5.1 |
| *Micrococcus luteus* | 49732 | pos | N/A | 2.8 | 4 | >5.0 |
| *Streptococcus mutans* | 25175 | pos | N/A | 3.4 | 4.4 | >5.5 |
| *Streptococcus pneumoniae* | 10015 | pos | N/A | 2.5 | 3.6 | >5.0 |
| *Corynebacterium diphtheriae* | 296 | pos | N/A | 1.6 | >4.5 | 1.5 |
| *Corynebacterium tuberculostearicum* | 35693 | pos | N/A | 1.6 | >5.1 | 3.9 |

### Example 4

### Cytotoxicity

Formulations 14 and 16 were either E-beam or gamma sterilized and were evaluated for potential cytotoxic effects following ISO Guideline 10993-5, Biological Evaluation of Medical Devices - Part 5: Tests for *in vitro* Cytotoxicity. Formulations 14 and 15 were extracted in purified water (PW) at 37° C for 24 hours. The PW extract was mixed with double strength Minimum Essential Medium (2X MEM) to a 50% concentration. A negative control (high density polyethylene) and reagent control (*e.g.,* PW) were similarly prepared. A positive control (powder-free latex gloves which include natural rubber latex, zinc carbamate accelerators, zinc oxide and titanium dioxide) was extracted in single strength MEM (IX MEM) at 37° C for 24 hours. Triplicates of a mammalian cell culture monolayer having L-929 mouse fibroblast cells were dosed with each extract (formulations 14, 16, positive, negative and reagent controls) and incubated at 37° C in the presence of 5% CO₂ for 48 hours. Following incubation, the monolayers were examined microscopically (100X) for abnormal cell morphology and cellular degeneration.

To confirm the scores, 0.2 ml of trypan blue stain was added to wells containing the test samples. The trypan blue molecule is large and cannot readily be absorbed by live cells. Only dead cells or those with compromised cell membranes take up the blue colored stain. Table 6 describes the scoring and visual characteristics.

**Table 6**

| **Grade/Score** | **Reactivity** | **Conditions of all Cultures** |
|---|---|---|
| 0 | None | Discrete intracytoplasmic granules, no cell lysis, no reduction of cell growth. |
| 1 | Slight | Not more than 20% of the cells are round, loosely attached and without intracytoplasmic granules, or show changes in morphology; occasional lysed cells are present; only slight growth inhibition observable. |
| 2 | Mild | Not more than 50% of the cells are round, devoid of intracytoplasmic granules; no extensive cell lysis; not more than 50% growth inhibition observable. |
| 3 | Moderate | Not more than 70% of the cell layers contain rounded cells or are lysed; cell layers not completely destroyed, but more than 50% growth inhibition observed. |
| 4 | Severe | Nearly complete or complete destruction of the cell layers. |

Set out below in Table 7 are the results for either E-beam (e) or gamma (g) sterilized versions of Formulations 14 and 16, with the subscripts e or g denoting the sterilization method:

**Table 7**

| **Cytotoxicity** | | | | | |
|---|---|---|---|---|---|
| **Sample** | **Percent Rounding** | **Percent Cells Without Intracytoplasmic Granules** | **Percent Lysis** | **Grade** | **Reactivity** |
| Formulation 14ₑ | 0 | 0 | 0 | 0 | None |
| Formulation 14_{g} | 0 | 0 | 0 | 0 | None |
| Formulation 16ₑ | 10 | 10 | 10 | 1 | Slight |
| Formulation 16_{g} | 0 | 0 | 0 | 0 | None |
| Negative Control | 0 | 0 | 0 | 0 | None |
| Reagent Control | 0 | 0 | 0 | 0 | None |
| Positive Control | Not Applicable | Not Applicable | 100 | 4 | Severe |

Formulation 16ₑ showed slight cell lysis or toxicity but is generally considered to be non-cytotoxic with a cytoxicity score of 1. Formulations 16_{g}, 14ₑ and 14_{g} were shown to be nontoxic with each having a cytoxicity score of 0.

### Example 5

### Ciliotoxicity in Rabbits

Formulations 14 and 16 were either E-beam or gamma sterilized and evaluated for their effect on rabbit cilia. A 0.6 cc portion of each formulation (Formulations 14, 16 and control (no treatment, saline only)) were applied to the bilateral maxillary sinus antrostomies of randomized New Zealand White rabbits using a single lumen catheter. Following treatment, daily irrigation with 3 cc of saline was performed. At the end of 7 or 10 days, the rabbits were euthanized, and a piece of the medial wall of the maxillary sinuses was removed and fixed (Karnovsky's fixture) for scanning electron microscopy (SEM).

In a blind analysis, the tissues samples were evaluated and scored for the presence or absence of cilia. The results are shown in Table 8.

**Table 8**

| **Ciliotoxicity in Rabbits** | | |
|---|---|---|
| **Formulation** | **E-beam** | **Gamma** |
| **Saline control** | Cilia present | Cilia present |
| **14** | Cilia present | Cilia present |
| **16** | Cilia present | Cilia present |

Rabbits treated with the formulations showed the presence of cilia similar to the saline control. These results suggest that these formulations were non-toxic to cilia. In addition, the sterilization method did not appear to affect the formulation's toxicity towards cilia, namely both gamma and E-beam sterilized formulations were non-toxic to cilia.

### Example 6

### Ciliotoxicity in Sheep

Endoscopic sinus surgery including bilateral middle turbinectomy and partial thickness wounding around the ethmoidal cell was performed on sheep. The sheep were randomized and one nare was treated with 7 ml of each test formulation (formulation 7 and 8), the other nare with no treatment (control). Daily bilateral 20cc saline irrigations were performed through the nose. Twenty-eight days after the surgery, two pieces of the medial wall of the nasal/sinus cavity were obtained from each sheep and each piece was fixed either for SEM analysis (Karnovsky's fixative) or histology or pathology evaluation (formalin). In a blind analysis, the tissue samples were evaluated and scored for the presence or absence of cilia.

No paste material was observed in the sheep after 28 days. SEM analysis showed cilia were present in both treated and control samples. Microscopic evaluation revealed similar inflammatory response between the treated samples and control. These results suggest that the formulations are safe for use *in vivo.*

### Example 7

### Sensitization and Irritation

Formulation 16 was evaluated for sensitization using a guinea pig model and ISO Guideline 10993-10, Biological Evaluation of Medical Devices - Part 10: Tests for irritation and skin sensitization. The formulation received sensitization scores of 0 for all test sites in a guinea pig model, and did not show any evidence of a delayed reaction. The formulation also received an irritation index of 0 using microscopic evaluation of vaginal mucosal tissue in a rabbit model. The formulation would not be considered a sensitizer or an irritant under the ISO Guideline.

Formulation 16 was also evaluated for sensitization using an ELISA Protein Analysis. The formulation was found to contain 5.1 ppm shellfish allergen or 0.00051% shellfish protein, with a 2 ppm limit of detection.

### Example 8

### Acute Systemic Toxicity

Formulation 16 was evaluated for acute systemic toxicity using a rat model, a 5 g/Kg oral route dose and ISO Guideline 10993-11, Biological Evaluation of Medical Devices - Part 11: Tests for systemic toxicity. For a packaged product for use in adult humans containing about 12 g of the disclosed paste, the chosen 5/g/kg test dose would represent about 29 times the adult dose. All animals survived the study.

### Example 9

### Residence Time

Formulation 16 was evaluated to determine its residence time and the formation of adhesions following application to wounded tissues in the nasal cavities of ten adult sheep. All animals in the study tolerated the procedure well and nine displayed a normal post-operative recovery with minimal adverse events. The observed residence time was 7 to 14 days and no adhesions were present in any of the animals during the study.

## Claims

1. A sinus stent paste comprising a water-soluble chitosan polymer or derivative thereof in which one or more hydroxyl or amine groups of the polymer have been modified to alter the solubility or mucoadhesion characteristics of the derivative and an osmolality reducing agent dissolved in a phosphate-containing solution to provide an opaque paste at 20 to 25°C that contains 5 to 20 wt.% of the water-soluble chitosan polymer or derivative thereof, and has a pH of at least 4, a viscosity of 1 to 15 Pa.s. measured according to ASTM F2103-11, Part 5, an osmolality of 270 to 2000 mOsm/kg, and a residence time of at least 1 day, wherein the osmolality reducing agent does not crosslink with the water-soluble chitosan polymer or derivative thereof.

2. A method for preparing a chitosan stenting paste comprising the steps of:
mixing and dissolving a water-soluble chitosan polymer or derivative thereof in which one or more hydroxyl or amine groups of the polymer have been modified to alter the solubility or mucoadhesion characteristics of the derivative and an osmolality reducing agent in a phosphate-containing solution to provide an opaque paste at 20 to 25°C that contains 5 to 20 wt.% of the water-soluble chitosan polymer or derivative thereof, and has a pH of at least 4, a viscosity of 1 to 15 Pa.s. measured according to ASTM F2103-11, Part 5, an osmolality of 270 to 2000 mOsm/kg, and a residence time of at least 1 day, wherein the osmolality reducing agent does not crosslink with the water-soluble chitosan polymer or derivative thereof.

3. A paste composition for use in stenting internal tissue or an internal surgical site, the composition comprising a water-soluble chitosan polymer or derivative thereof in which one or more hydroxyl or amine groups of the polymer have been modified to alter the solubility or mucoadhesion characteristics of the derivative and an osmolality reducing agent dissolved in a phosphate-containing solution to provide an opaque paste at 20 to 25°C that contains 5 to 20 wt.% of the water-soluble chitosan polymer or derivative thereof, and has a pH of at least 4, a viscosity of 1 to 15 Pa.s. measured according to ASTM F2103- 11, Part 5, an osmolality of 270 to 2000 mOsm/kg, and a residence time of at least 1 day, wherein the osmolality reducing agent does not crosslink with the water-soluble chitosan polymer or derivative thereof.

4. A paste, method or paste composition according to any preceding claim wherein the water-soluble chitosan polymer is from a chloride, citrate, nitrate, lactate, phosphate, or glutamate salt.

5. A paste, method or paste composition according to any preceding claim wherein the water-soluble chitosan polymer or derivative thereof is 12 wt. % to 18 wt. % of the total paste weight.

6. A paste, method or paste composition according to any preceding claim wherein the water-soluble chitosan polymer or derivative thereof has a number average molecular weight of 5 to 2000 kDa.

7. A paste, method or paste composition according to any preceding claim wherein the osmolality reducing agent comprises a hydroxyl-functional or alkyl-modified cellulose selected from methylcellulose, ethylcellulose, hydroxybutyl methylcellulose, hydroxyethylcellulose, hydroxypropylcellulose, hydroxypropyl methylcellulose, carboxymethylcellulose, and mixtures thereof.

8. The paste, method or paste composition according to claim 7 wherein the hydroxyl-functional or alkyl-modified cellulose is hydroxypropyl cellulose, methyl cellulose or hydroxyethyl cellulose.

9. A paste, method or paste composition according to any preceding claim wherein the osmolality reducing agent is 1 to 20 wt. % of the total paste weight.

10. A paste, method or paste composition according to any preceding claim wherein the phosphate-containing solution is a phosphate-buffered saline having a pH of 9 to 12.

11. A paste, method or paste composition according to any preceding claim wherein the paste is in sterilized packaging and packaged in a ready-to-use, storage stable, injectable or extrudable form, requiring no or minimal mixing, stirring, hydration or other preparation.

12. A paste, method or paste composition according to any preceding claim having an adhesive strength of between 5 grams and 80 grams (0.05 N to 0.78 N) of force required when using a tensile testing machine operated at a separation rate of 1mm/s to separate two collagen-coated rubber hemispheres that have been compressed against one another, with a sample of the paste between them, using a 4.4 Newton compression force.

13. A paste, method or paste composition according to any preceding claim wherein the paste is non-cytotoxic with a cytotoxicity score of 1 or less as measured by ISO Guideline 10993-5.

14. A paste, method or paste composition according to any preceding claim wherein the phosphate-containing solution comprises a glycerol phosphate.

15. A paste, method or paste composition according to any preceding claim wherein the paste further comprises a lubricant or wetting agent and the lubricant or wetting agent is 1 to 10 wt. % of the total paste weight.

## Patentansprüche

1. Sinus-Stenting-Paste, die ein wasserlösliches Chitosanpolymer oder ein Derivat davon, wobei eine oder mehrere Hydroxyl- oder Aminogruppen des Polymers modifiziert worden sind, um die Löslichkeits- oder Mukoadhäsionscharakteristika des Derivats zu ändern, und ein Osmolalitätsreduktionsmittel umfasst, die in einer phosphathaltigen Lösung gelöst sind, um eine opake Paste bei 20 bis 25 °C bereitzustellen, die 5 bis 20 Gew.% des wasserlöslichen Chitosanpolymers oder Derivats davon enthält, und einen pH-Wert von mindestens 4, eine Viskosität von 1 bis 15 Pa·s, gemessen gemäß ASTM F2103-11, Teil 5, eine Osmolalität von 270 bis 2000 mOsm/kg und eine Verweilzeit von mindestens 1 Tag aufweist, wobei das Osmolalitätsreduktionsmittel mit dem wasserlöslichen Chitosanpolymer oder Derivat davon nicht vernetzt.A

2. Verfahren zum Herstellen einer Chitosan-Stenting-Paste, umfassend die Schritte:
Mischen und Lösen eines wasserlöslichen Chitosanpolymers oder eines Derivats davon, wobei eine oder mehrere Hydroxyl- oder Aminogruppen des Polymers modifiziert worden sind, um die Löslichkeits- oder Mukoadhäsionscharakteristika des Derivats zu ändern, und eines Osmolalitätsreduktionsmittels in einer phosphathaltigen Lösung, um eine opake Paste bei 20 bis 25 °C bereitzustellen, die 5 bis 20 Gew.% des wasserlöslichen Chitosanpolymers oder Derivats davon enthält, und einen pH-Wert von mindestens 4, eine Viskosität von 1 bis 15 Pa·s, gemessen gemäß ASTM F2103-11, Teil 5, eine Osmolalität von 270 bis 2000 mOsm/kg und eine Verweilzeit von mindestens 1 Tag aufweist, wobei das Osmolalitätsreduktionsmittel nicht mit dem wasserlöslichen Chitosanpolymer oder Derivat davon vernetzt.

3. Pastenzusammensetzung zur Verwendung beim Stenting von innerem Gewebe oder einem inneren Operationssitus, wobei die Zusammensetzung ein wasserlösliches Chitosanpolymer oder ein Derivat davon, wobei eine oder mehrere Hydroxyl- oder Aminogruppen des Polymers modifiziert worden sind, um die Löslichkeits- oder Mukoadhäsionscharakteristika des Derivats zu ändern, und ein Osmolalitätsreduktionsmittel gelöst in einer phosphathaltigen Lösung umfasst, um eine opake Paste bei 20 bis 25 °C bereitzustellen, die 5 bis 20 Gew.% des wasserlöslichen Chitosanpolymers oder Derivats davon enthält, und einen pH-Wert von mindestens 4, eine Viskosität von 1 bis 15 Pa·s, gemessen gemäß ASTM F2103-11, Teil 5, eine Osmolalität von 270 bis 2000 mOsm/kg und eine Verweilzeit von mindestens 1 Tag aufweist, wobei das Osmolalitätsreduktionsmittel nicht mit dem wasserlöslichen Chitosanpolymer oder Derivat davon vernetzt.

4. Paste, Verfahren oder Pastenzusammensetzung nach einem der vorhergehenden Ansprüche, wobei das wasserlösliche Chitosanpolymer von einem Chlorid-, Citrat-, Nitrat-, Lactat-, Phosphat- oder Glutamatsalz ist.

5. Paste, Verfahren oder Pastenzusammensetzung nach einem der vorhergehenden Ansprüche, wobei das wasserlösliche Chitosanpolymer oder Derivat davon 12 Gew.% bis 18 Gew.% des gesamten Pastengewichts ausmacht.

6. Paste, Verfahren oder Pastenzusammensetzung nach einem der vorhergehenden Ansprüche, wobei das wasserlösliche Chitosanpolymer oder Derivat davon ein zahlenmittleres Molekulargewicht von 5 bis 2000 kDa aufweist.

7. Paste, Verfahren oder Pastenzusammensetzung nach einem der vorhergehenden Ansprüche, wobei das Osmolalitätsreduktionsmittel eine hydroxylfunktionale oder alkylmodifizierte Cellulose ausgewählt aus Methylcellulose, Ethylcellulose, Hydroxybutylmethylcellulose, Hydroxyethylcellulose, Hydroxypropylcellulose, Hydroxypropylmethylcellulose, Carboxymethylcellulose und Mischungen davon umfasst.

8. Paste, Verfahren oder Pastenzusammensetzung nach Anspruch 7, wobei die hydroxylfunktionale oder alkylmodifizierte Cellulose Hydroxylpropylcellulose, Methylcellulose oder Hydroxyethylcellulose ist

9. Paste, Verfahren oder Pastenzusammensetzung nach einem der vorhergehenden Ansprüche, wobei das Osmolalitätsreduktionsmittel 1 Gew.% bis 20 Gew.% des gesamten Pastengewichts ausmacht.

10. Paste, Verfahren oder Pastenzusammensetzung nach einem der vorhergehenden Ansprüche, wobei die phosphathaltige Lösung eine phosphatgepufferte Kochsalzlösung mit einem pH-Wert von 9 bis 12 ist.

11. Paste, Verfahren oder Pastenzusammensetzung nach einem der vorhergehenden Ansprüche, wobei die Paste in einer sterilisierten Verpackung vorliegt und in einer gebrauchsfertigen, lagerstabilen, injizierbaren oder extrudierbaren Form verpackt ist, die kein oder minimales Mischen, Rühren, Hydratisieren oder sonstiges Vorbereiten erfordert.

12. Paste, Verfahren oder Pastenzusammensetzung nach einem der vorhergehenden Ansprüche mit einer Haftfestigkeit zwischen 5 Gramm Kraft und 80 Gramm Kraft (0,05 N bis 0,78 N), die benötigt wird, wenn eine Zugprüfmaschine, die mit einer Trennrate von 1 mm/s betrieben wird, zum Trennen von zwei kollagenbeschichteten Kautschukhalbkugeln verwendet wird, die unter Verwendung einer Kompressionskraft von 4,4 Newton gegeneinander komprimiert worden sind, wobei sich eine Probe der Paste dazwischen befindet.

13. Paste, Verfahren oder Pastenzusammensetzung nach einem der vorhergehenden Ansprüche, wobei die Paste nicht zytotoxisch ist und eine Zytotoxizitätsbewertung von 1 oder weniger aufweist, gemessen gemäß der ISO-Richtlinie 10993-5.

14. Paste, Verfahren oder Pastenzusammensetzung nach einem der vorhergehenden Ansprüche, wobei die phosphathaltige Lösung ein Glycerinphosphat umfasst.

15. Paste, Verfahren oder Pastenzusammensetzung nach einem der vorhergehenden Ansprüche, wobei die Paste ferner ein Schmiermittel oder Benetzungsmittel umfasst, und das Schmiermittel oder Benetzungsmittel 1 Gew.% bis 10 Gew.% des gesamten Pastengewichts ausmacht.

## Revendications

1. Pâte d'endoprothèse de sinus comprenant un polymère de chitosane hydrosoluble ou un dérivé de celui-ci dans lequel un ou plusieurs groupes hydroxyle ou aminé du polymère ont été modifiés pour altérer les caractéristiques de solubilité ou de mucoadhésion du dérivé et un agent réduisant l'osmolalité dissous dans une solution contenant du phosphate pour fournir une pâte opaque à 20 à 25 °C qui contient de 5 à 20 % en poids du polymère de chitosane hydrosoluble ou de son dérivé, et a un pH d'au moins 4, une viscosité de 1 à 15 Pa.s., mesurée selon l'ASTM F2103-11, section 5, une osmolalité de 270 à 2000 mOsm/kg et un temps de séjour d'au moins 1 jour, dans laquelle l'agent réducteur de l'osmolalité ne réticule pas avec le polymère de chitosane hydrosoluble ou son dérivé.

2. Procédé de préparation d'une pâte d'occlusion au chitosane comprenant les étapes consistant à :
mélanger et dissoudre un polymère de chitosane hydrosoluble ou un dérivé de celui-ci dans lequel un ou plusieurs groupes hydroxyle ou amine du polymère ont été modifiés pour altérer les caractéristiques de solubilité ou de mucoadhésion du dérivé et un agent réduisant l'osmolalité dissous dans une solution contenant du phosphate pour fournir une pâte opaque à 20 à 25 °C qui contient de 5 à 20 % en poids du polymère de chitosane hydrosoluble ou de son dérivé, et a un pH d'au moins 4, une viscosité de 1 à 15 Pa.s., mesurée selon l'ASTM F2103-11, section 5, une osmolalité de 270 à 2000 mOsm/kg et un temps de séjour d'au moins 1 jour, dans laquelle l'agent réducteur de l'osmolalité ne réticule pas avec le polymère de chitosane hydrosoluble ou son dérivé.

3. Composition de pâte pour une utilisation dans l'occlusion de tissu interne ou d'un site chirurgical interne, la composition comprenant un polymère de chitosane hydrosoluble ou un dérivé de celui-ci dans lequel un ou plusieurs groupes hydroxyle ou amine du polymère ont été modifiés pour altérer les caractéristiques de solubilité ou de mucoadhésion du dérivé et un agent réduisant l'osmolalité dissous dans une solution contenant du phosphate pour fournir une pâte opaque à 20 à 25 °C qui contient de 5 à 20 % en poids du polymère de chitosane hydrosoluble ou de son dérivé, et a un pH d'au moins 4, une viscosité de 1 à 15 Pa.s., mesurée selon l'ASTM F2103-11, section 5, une osmolalité de 270 à 2000 mOsm/kg et un temps de séjour d'au moins 1 jour, dans laquelle l'agent réducteur de l'osmolalité ne réticule pas avec le polymère de chitosane hydrosoluble ou son dérivé.

4. Pâte, procédé ou composition de pâte selon l'une quelconque des revendications précédentes, dans laquelle le polymère de chitosane hydrosoluble provient d'un sel de chlorure, de citrate, de nitrate, de lactate, de phosphate ou de glutamate.

5. Pâte, procédé ou composition de pâte selon l'une quelconque des revendications précédentes, dans laquelle le polymère de chitosane hydrosoluble ou son dérivé représente de 12 % en poids à 18 % en poids du poids total de la pâte.

6. Pâte, procédé ou composition de pâte selon l'une quelconque des revendications précédentes, dans laquelle le polymère de chitosane hydrosoluble ou son dérivé a un poids moléculaire moyen en nombre de 5 à 2000 kDa.

7. Pâte, procédé ou composition de pâte selon l'une quelconque des revendications précédentes, dans laquelle l'agent réduisant l'osmolalité comprend une cellulose à fonctionnalité hydroxyle ou modifiée par un alkyle choisie parmi la méthylcellulose, l'éthylcellulose, l'hydroxybutylméthylcellulose, l'hydroxyéthylcellulose, l'hydroxypropylcellulose, l'hydroxypropylméthylcellulose, la carboxyméthylcellulose et leurs mélanges.

8. Pâte, procédé ou composition de pâte selon la revendication 7, dans laquelle la cellulose à fonctionnalité hydroxyle ou modifiée par un groupe alkyle est l'hydroxypropylcellulose, la méthylcellulose ou l'hydroxyéthylcellulose.

9. Pâte, procédé ou composition de pâte selon l'une quelconque des revendications précédentes, dans laquelle l'agent réduisant l'osmolalité représente de 1 et 20 % en poids du poids total de la pâte.

10. Pâte, procédé ou composition de pâte selon l'une quelconque des revendications précédentes, dans laquelle la solution contenant du phosphate est une solution saline tamponnée au phosphate ayant un pH de 9 à 12.

11. Pâte, procédé ou composition de pâte selon l'une quelconque des revendications précédentes, dans laquelle la pâte est conditionnée dans un emballage stérilisé et emballée sous une forme prête à l'emploi, stable au stockage, injectable ou extrudable, ne nécessitant pas ou peu de mélange, d'agitation, d'hydratation ou autre préparation.

12. Pâte, procédé ou composition de pâte selon l'une quelconque des revendications précédentes, ayant une force adhésive comprise entre 5 grammes et 80 grammes (0,05 N à 0,78 N) de force requise lors de l'utilisation d'une machine de test de traction fonctionnant à une vitesse de séparation de 1 mm/s pour séparer deux hémisphères en caoutchouc recouverts de collagène qui ont été comprimés l'un contre l'autre, avec un échantillon de la pâte entre eux, avec une force de compression de 4,4 Newton.

13. Pâte, procédé ou composition de pâte selon l'une quelconque des revendications précédentes, dans laquelle la pâte est non cytotoxique avec un score de cytotoxicité de 1 ou moins, tel que mesuré par la directive ISO 10993-5.

14. Pâte, procédé ou composition de pâte selon l'une quelconque des revendications précédentes, dans laquelle la solution contenant du phosphate comprend un phosphate de glycérol.

15. Pâte, procédé ou composition de pâte selon l'une quelconque des revendications précédentes, dans laquelle la pâte comprend en outre un lubrifiant ou un agent mouillant et le lubrifiant ou l'agent mouillant représente de 1 à 10 % en poids du poids total de la pâte.
